# EUROPEAN PATENT APPLICATION

(11) **EP 4 830 644 A1**
(43) Date of publication of application: **28.10.2026**
(21) Application number: 26174793.5
(22) Date of filing: 27.04.2026
(51) Int. Cl.: A61B 17/072, A61B 17/00, A61B 17/29, A61B 90/00

(54) **SURGICAL SYSTEM**

(30) Priority: 25.04.2025 US 202519190184
(71) Applicant: Cilag GmbH International, 6300 Zug (CH)
(72) Inventor: MAYANS, Joan Aguilar, 6300 Zug (CH); VON STEIN, Jonathan Zachary, 6300 Zug (CH); GOSSELL, Jordan Christopher, 6300 Zug (CH)
(74) Representative: Carpmaels & Ransford LLP

(57) **Abstract**

A surgical system for use with a surgical instrument including an end effector articulatable relative to a shaft of the surgical instrument . The surgical system includes a control circuit that receives an input signal indicative of a desired articulation position of the end effector, generates a drive signal for articulation of the end effector based on the input signal to the desired articulation position, and actively maintains the end effector at the desired articulation position based on a parameter independent of the articulation of the end effector.

## Description

### BACKGROUND

Surgical stapling instruments are commonly used in procedures involving tissue transection and staple formation. In such devices, the end effector is often designed to articulate relative to a surgical instrument's shaft to optimize access and positioning.

During tissue transection and staple formation, significant internal forces are generated due to the firing process. These forces can lead to unintended movement of the end effector-referred to as de-articulation-which may compromise the accuracy of the surgical procedure, negatively impact patient outcomes, and result in a less favorable user experience.

Conventional hardware-based methods to counteract these forces add complexity and reduce controllability, making them less desirable in modern, highly dynamic surgical systems.

### SUMMARY

In one aspect, the present disclosure provides a surgical system for use with a surgical instrument including an end effector articulatable relative to a shaft of the surgical instrument . The surgical system includes a control circuit that receives an input signal indicative of a desired articulation position of the end effector, generates a drive signal for articulation of the end effector based on the input signal to the desired articulation position, and actively maintains the end effector at the desired articulation position based on a parameter independent of the articulation of the end effector.

In one aspect, the present disclosure provides a surgical system for use with a surgical instrument including an end effector articulatable relative to a shaft of the surgical instrument. The surgical instrument includes a control circuit configured to: receive an input signal indicative of a desired articulation position of the end effector, generate a drive signal for articulation of the end effector based on the input signal to the desired articulation position, receive a first sensor signal indicative of a knife input torque associated with advancing a knife relative to the end effector through a tissue transection path, receive a second sensor signal indicative of a position of the knife along the tissue transection path, and actively maintain the end effector at the desired articulation position based on the knife input torque and the position of the knife.

In one aspect, the present disclosure provides a surgical system including a surgical instrument that may include: a shaft; an articulation joint extending from the shaft; an end effector articulatable about the articulation joint relative to the shaft; and a firing assembly, may include a knife movable to cut tissue along a transection path defined by the end effector. The system also includes a control circuit configured to: receive a first sensor signal indicative of a knife input torque associated with advancing the knife relative to the end effector along the transection path, and generate an articulation control signal to actively maintain the end effector at a desired articulation position based on the knife input torque.

### BRIEF DESCRIPTION OF THE DRAWINGS

In the description, for purposes of explanation and not limitation, specific details are set forth, such as particular aspects, procedures, techniques, etc. to provide a thorough understanding of the present technology. However, it will be apparent to one skilled in the art that the present technology may be practiced in other aspects that depart from these specific details.

The accompanying drawings, where like reference numerals refer to identical or functionally similar elements throughout the separate views, together with the detailed description below, are incorporated in and form part of the specification, and serve to further illustrate aspects of concepts that include the claimed disclosure and explain various principles and advantages of those aspects.

The systems and instruments disclosed herein have been represented where appropriate by conventional symbols in the drawings, showing only those specific details that are pertinent to understanding the various aspects of the present disclosure so as not to obscure the disclosure with details that will be readily apparent to those of ordinary skill in the art having the benefit of the description herein.
FIG. 1 is a schematic view of a surgical system for use with a surgical instrument, in accordance with the disclosed technology.
FIG. 1A is a schematic view of an end effector of the surgical instrument of FIG. 1.
FIG. 2 a logic diagram depicting a method, in accordance with the disclosed technology.
FIG. 3 is a logic diagram depicting a method, in accordance with the disclosed technology.
FIG. 4 is a logic diagram depicting a method, in accordance with the disclosed technology.
FIG. 5 is a logic diagram depicting a method, in accordance with the disclosed technology.
FIG. 6 is a graph that illustrates a de-articulation correction output during firing and retraction, in accordance with the disclosed technology.
FIG. 7 is a schematic perspective view of a surgical instrument that includes a surgical instrument, in accordance with the disclosed technology;
FIG. 7A is a schematic detail view of one side of a housing, depicting rotational pucks that engage a robotic platform, in accordance with the disclosed technology.
FIG. 8 is a schematic detail view of an end effector, articulation joint, and portions of a cable articulation assembly, firing assembly, and roll assembly, in accordance with the disclosed technology;
FIG. 9 is a schematic detail view of the end effector at a desired articulation position with respect to and the articulation joint, in accordance with the disclosed technology;
FIG. 10 is a schematic exploded view of a distal end of the surgical instrument, in accordance with the disclosed technology; and
FIG. 11 is a schematic detail view of a knife, in accordance with the disclosed technology.

### DESCRIPTION

During tissue transection and staple formation, the firing mechanism of the stapling device generates large internal forces. These forces often cause the articulating end effector to move involuntarily toward a neutral "home" position-a phenomenon known as de-articulation. The resulting unintended movement can impair surgical precision, lead to suboptimal patient outcomes, and reduce surgeon confidence in the device.

The present disclosure describes a surgical system that implements a compensation method that actively maintains the desired articulation of the end effector despite the internal forces generated during transection and staple formation. The system monitors a parameter, also referred to herein as "first parameter", independent of the articulation and uses this real-time data to compute an additional articulation command. This command may be gradually ramped up at the beginning of the transection and gradually ramped down as retraction ends, which provides an effective correction to counteract de-articulation.

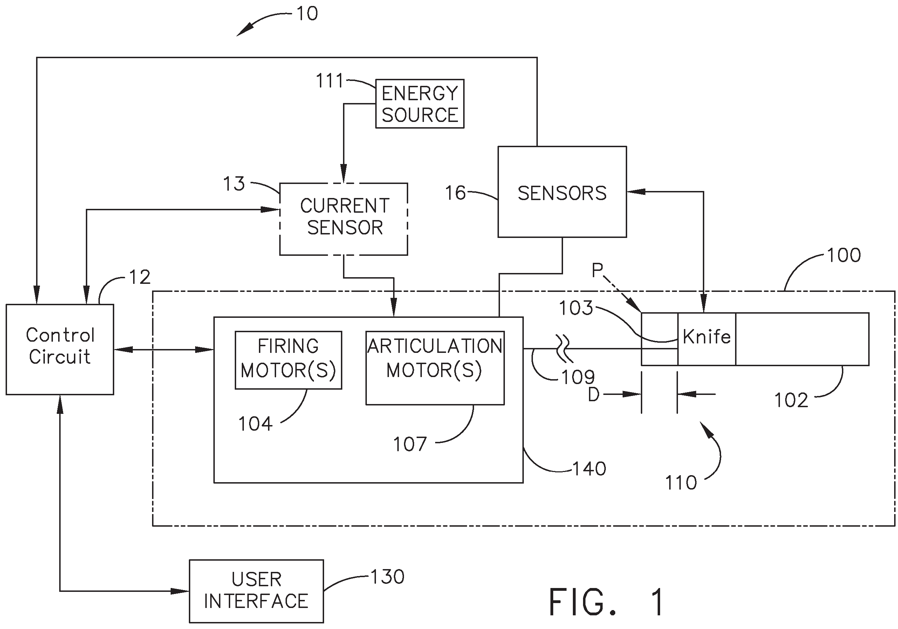

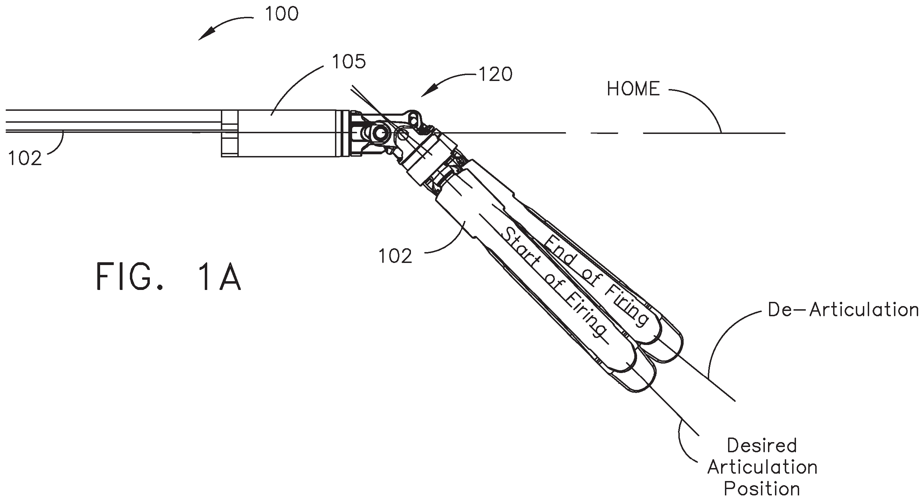

FIG. 1 depicts a surgical system 10 that may be used with a surgical instrument 100 (e.g., robotic surgical instrument or handheld surgical instrument). The surgical instrument 100 may include an end effector 102 articulatable relative to a shaft 105 of the surgical instrument 100.

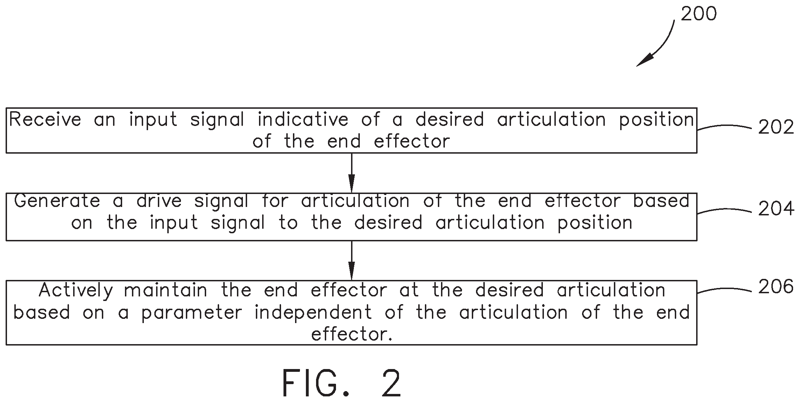

The surgical system 10 includes a control circuit 12 that executes one or more parts of a method 200 (FIG. 2). The method 200 includes receiving 202 an input signal indicative of a desired articulation position of the end effector 102. The input signal can be based on a user input received through a user interface 130. In at least one embodiment, the surgical instrument is a handheld instrument including a handle with actuators that can be moved by the user to control articulation of the end effector 102. Alternatively, the surgical instrument 100 may be a robotic surgical instrument including a robotic user interface that can be manipulated by the user to control articulation of the end effector.

The method 200 further includes generating 204 a drive signal for articulation of the end effector based on the input signal to the desired articulation position, and actively maintaining 206 the end effector 102 at the desired articulation position based on a parameter independent of the articulation of the end effector 102. In at least one embodiment, the parameter is associated with a function of the surgical instrument 100 separate from the articulation, or articulation data. Additionally, or alternatively, the parameter may be associated with a tissue treatment by the surgical instrument 100. Additionally, or alternatively, the parameter may be associated with a tissue stapling and/or transecting by the surgical instrument 100. Additionally, or alternatively, the parameter may be associated with one or more components of a firing assembly 110 of the surgical instrument 100, as described in greater detail below.

To actively maintain the end effector 102 at the desired articulation, the control circuit 12 may dynamically compensate for an unintended de-articulation from the desired articulation position during a tissue treatment by the end effector 102, as illustrated in FIG. 1A. The dynamic compensation may be implemented based on measured values of the parameter that is independent of the articulation or articulation data.

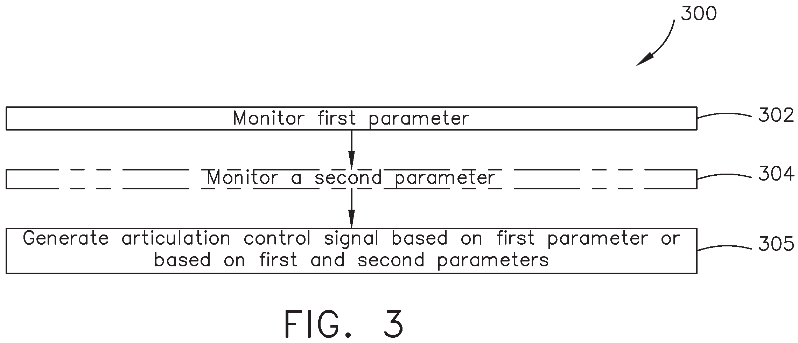

In at least one embodiment, the dynamic compensation may involve multiple real-time adjustments based on fluctuations in the measured values of the parameter. As illustrated in FIG. 3, the control circuit 12 may execute a method 300 that monitors 302 the parameter, and generates 306 an articulation control signal to dynamically adjust the articulation of the end effector 102 based on the monitored parameter. These adjustments may be executed over a defined time interval during tissue treatment, ensuring that the end effector 102 remains at the desired articulation position throughout the procedure. Additionally, or alternatively, the adjustments may be triggered based on a defined distance traveled by one or more components of the surgical instrument, such as one or more components of the firing assembly 110.

The adjustments may be applied continuously, where every detected change in the monitored parameter results in a corresponding articulation correction in real time. Alternatively, the adjustments may be applied intermittently, with parameter values sampled at predefined time intervals or at a specified frequency, allowing for discrete corrections that maintain stability while minimizing unnecessary fluctuations. The frequency and timing of these adjustments may be dynamically optimized based on the nature of the tissue interaction, the characteristics of the surgical procedure, or predefined system thresholds.

In at least one embodiment, the parameter represents at least one of a torque, a force, or a load associated with one or more components of the firing assembly 110. These components may include, for example, a knife 103, a firing drive/rod 109, a firing motor 104, or any other suitable element of the firing assembly 110. In one embodiment, the parameter corresponds to the load on the firing assembly 110 during staple firing and/or tissue transection. In another embodiment, the parameter reflects the torque experienced by the firing motor 104 while firing staples and/or transecting tissue. Additionally, the parameter may indicate the force or load exerted on the knife 103 or firing drive/rod 109 during the firing sequence and/or tissue transection.

As illustrated in FIG. 1, a current sensor 13 may monitor the current draw of the firing motor 104 from an energy source 111. The measured current draw may be directly correlated to the torque applied by the firing motor 104, as well as the load experienced by the knife 103 or firing drive/rod 109. Based on this data, the control circuit 12 may dynamically adjust the articulation of the end effector 102 to actively maintain the articulation of the end effector 102 at the desired articulation based on the current draw by the firing motor 104.

Additionally, or alternatively, other sensors 16 may be incorporated to determine the parameter. These sensors may include, for example, load cells for direct force or load measurement, strain gauges to detect material deformation and infer force and torque, and piezoelectric sensors that generate an electrical charge in response to mechanical stress. Additionally, or alternatively, the sensors 16 may include torque sensors for direct torque measurement, capacitive sensors that detect deformation via changes in capacitance, and optical sensors that utilize light-based displacement measurements to correlate with force and pressure variations.

The parameter may be derived from readings obtained from a single sensor or from multiple sensors working in concert. For instance, in one embodiment a single sensor-such as a current sensor, load cell, or strain gauge-might provide a direct measurement of a specific aspect of the firing assembly's performance, such as torque or load. In other embodiments, the parameter is determined by integrating data from multiple sensors to offer a more comprehensive view of the forces at play.

When multiple sensors are employed, their individual readings can be combined using sensor fusion algorithms. This approach allows the system to correlate, validate, and refine the parameter measurement by incorporating diverse inputs. For example, data from a current sensor monitoring the firing motor 104 may be merged with strain gauge measurements on the knife 103, providing a composite parameter that more accurately reflects the overall mechanical stress during tissue transection and staple firing. Additionally, combining readings can introduce redundancy, thereby enhancing reliability by mitigating the risk of errors due to sensor drift or failure.

Moreover, the integration may involve weighting factors or digital filtering techniques, as described in greater detail below, ensuring that the final parameter value is both precise and robust. Whether the readings are taken continuously or at defined intervals, the resulting parameter is used to dynamically adjust the articulation of the end effector 102 to maintain the articulation at the desired articulation.

In at least one embodiment, a second parameter may be utilized in weighing or digital filtering the output of the control circuit 12 generated based on the first parameter. Like the first parameter, the second parameter may also be independent of the articulation of the end effector 102.

To actively maintain the end effector 102 at the desired articulation, the method 300 may further monitor 304 the second parameter and generate 306 the articulation control signal further based on the second parameter. The dynamic compensation or an unintended de-articulation from the desired articulation position during a tissue treatment by the end effector 102 may be implemented based on measured values of the first parameter that are weighed or filtered based on corresponding measured values of the second parameter.

The second parameter, like the first parameter, may be associated with a function of the surgical instrument separate from the articulation, or articulation data. Additionally, or alternatively, the second parameter may be associated with a tissue treatment by the surgical instrument. Additionally, or alternatively, the second parameter may be associated with a tissue stapling and/or transecting by the surgical instrument. Additionally, or alternatively, the second parameter may be associated with one or more components of the firing assembly 110 of the surgical instrument, as described in greater detail below.

In at least one embodiment, the second parameter indicates the distance (D) travelled by, or relative position (P) of, one or more components of the firing assembly 110 during the tissue treatment by the end effector 102. In one embodiment, the second parameter may represent the position (P) of the knife 103 or distance (D) travelled by the knife 103 during staple firing and/or transection, as illustrated in FIG. 1.

Readings from one or more sensors 16 can be used by the control circuit 12 to ascertain the second parameter. For example, the sensors 16 may include Linear Variable Differential Transformers (LVDTs) for high-accuracy linear displacement measurements, rotary encoders for angular position measurements of the firing motor 104, and potentiometers for linear or angular displacement measurements. Hall Effect sensors, which use magnetic fields for contactless measurement, capacitive sensors, inductive sensors, which detect position changes by measuring variations in capacitance or inductance, and/or optical sensors, may also be utilized.

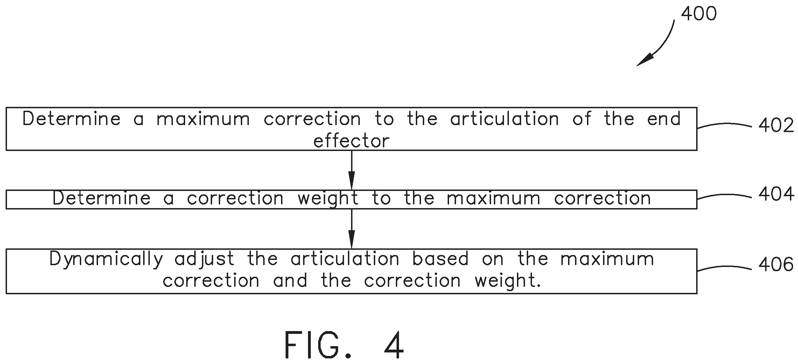

Referring now to FIG. 4, in at least one embodiment, to actively maintain the end effector 102 at the desired articulation, the control circuit 12 may execute a method 400 that determines 402 a maximum correction to the articulation of the end effector 102, determines 404 a correction weight to the maximum correction, and dynamically adjusts 406 the articulation based on the maximum correction and the correction weight. In at least one embodiment, the maximum correction (MC) may be determined based on the first parameter using an equation defined as: $MC = G ∗ A ∗ P 1$ where:
*G is a Gain value.*
*A is the articulation angle of the end ef fector.*
*P*1 *is the first parameter.*

Furthermore, the correction weight (CW) may be determined based on the second parameter using an equation defined as: $CW = MC ∗ \left(P2/T\right)$ where:
*MC is the maximum correction;*
*P2 is the second parameter;* and
*T is defined threshold.*

Additionally, the adjustments may be determined based on an equation defined as: $AJ = MC ∗ CW$ where:
*MC is the maximum correction;*
*CW is the correction weight;* and
*AJ is the adjustment.*

The correction weight (CW) facilitates a gradual increase, or decrease, to the correction of the articulation based on the second parameter, thereby improving the stability of the surgical instrument 100. In at least one embodiment, to actively maintain the end effector at the desired articulation, the control circuit may gradually increase, or gradually decrease, the correction to the articulation based on position, or distance travelled, by one or more components of the firing assembly 110 (e.g., knife, firing drive/rod, firing motor) during staple firing and/or transection.

In one embodiment, the control circuit may gradually increase the correction to the articulation for a first-time interval associated with a start of the tissue transection by the knife 103, for example, and/or may gradually decrease the correction for a second-time interval associated with an end of a retraction of the knife 103 relative to the end effector, for example.

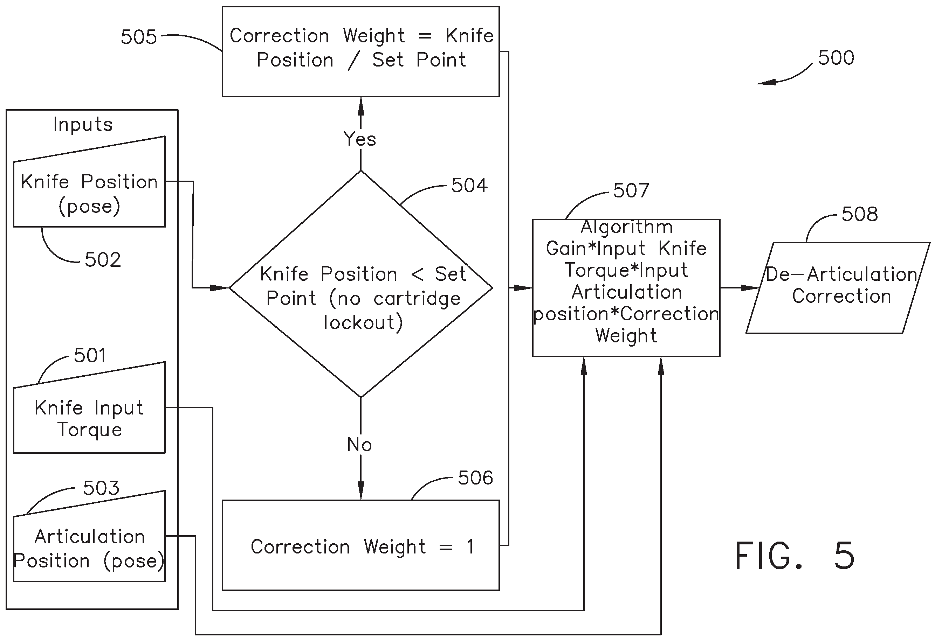

Referring now to FIG. 5, to move the end effector relative to the shaft to the desired articulation position, the control circuit may leverage the knife input torque 501 and the knife position 502. The control circuit 12 may obtain these values and the articulation position 503. The control circuit 12 may further utilize the knife position 502 as a weighing value by comparing 504 the knife position 502 readings to a determined set point, or threshold. As the knife 103 is gradually advanced along the transection path, the correction weight (CW) gradually increases (505, and its impact on the maximum correction (MC) gradually decreases until the correction weight (CW) reaches the value of 1 (506). The adjustments to the articulation of then effector may be determined 507 by an equation defined as: $AJ = G ∗ KT ∗ A ∗ CW$ where:
*G is a Gain value;*
*A is the input articulation Position;*
*CW is the correction weight;*
*KT is a knife input torque;* and
*AJ is the adjustment.*

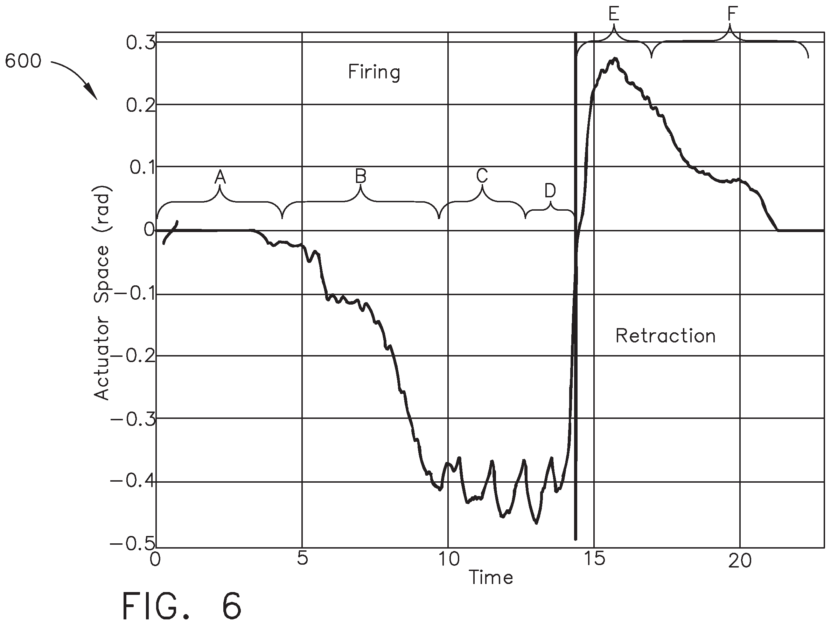

Further to the above, the control circuit 12 may generate an articulation control signal for one or more articulation motors 107 of a motor assembly 140 (FIG. 1) to implement 508 the de-articulation correction. FIG. 6 is a graph 600 that illustrates a de-articulation correction output during firing and retraction, in accordance with at least one embodiment of the present disclosure. In an initial segment (A) of the firing sequence, prior to engaging tissue, no de-articulation correction output is needed.

In a following segment (B) of the firing sequence, as staple firing and tissue transection commences, the load seen by the firing assembly 110 gradually increases. Consequently, the control circuit 12 correspondingly increases the de-articulation correction output to maintain the end effector 102 at the desired articulation position. Once maximum correction (MC) is reached, additional adjustments, as shown in segment (C), are performed to stabilize the end effector at the desired articulation position based on real-time fluctuations in the first parameter.

Toward the end of the firing sequence, the control circuit 12 may rapidly remove the de-articulation correction and may further apply a counter correction, as illustrated in segment (E), to stabilize the end effector now that the internal forces necessitating the correction are removed. The counter correction is then gradually reduced through retraction as the firing assembly 110 returns to a home, or starting, position, as shown in segment (F), thereby ensuring a smooth transition from active correction to baseline articulation.

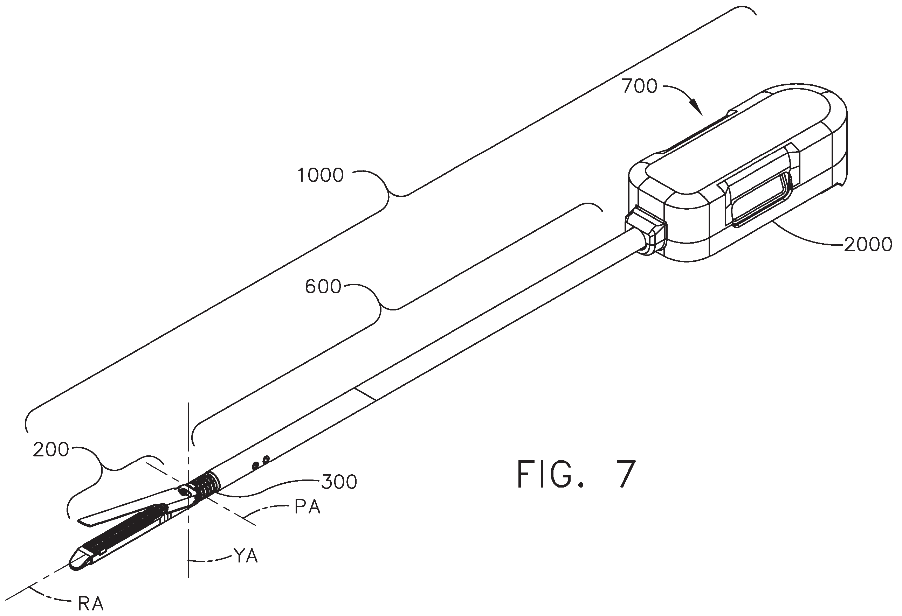

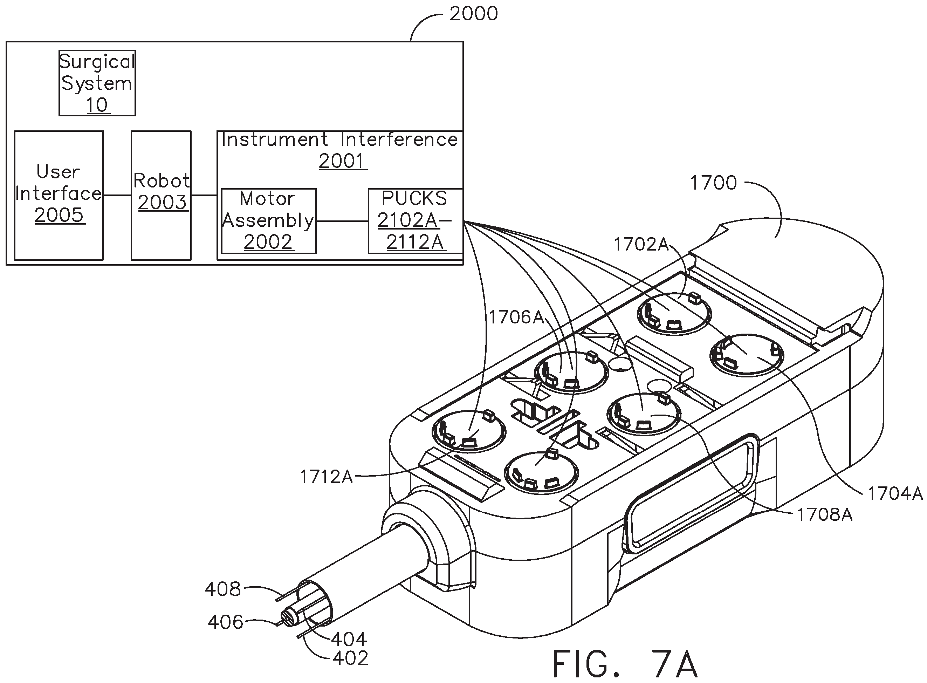

In some embodiments, as illustrated in FIG. 7A, the surgical system 10 may be used with, or integrated into, a robotic platform 2000 and/or a surgical instrument 1000 (FIG. 7) operable by the robotic platform 2000. Various details of the robotic platform 2000 and the surgical instrument 1000 are described in greater detail in United States Patent Application Serial No. 18/775,545, titled ROBOTIC STAPLING AND CUTTING SYSTEMS AND METHODS, filed July 17, 2024, which is hereby incorporated by reference herein in its entirety.

While the surgical instrument 10 incorporates a single-axis articulation joint 120, the surgical instrument 1000 is equipped with a multi-axis articulation joint 1300. Alternatively, the surgical instrument 10 may be equipped with a multi-axis articulation joint and/or the surgical instrument 1000 may be equipped with a single-axis articulation. Articulation adjustments by the surgical system 10 can be readily tailored to single-axis and muti-axis articulation.

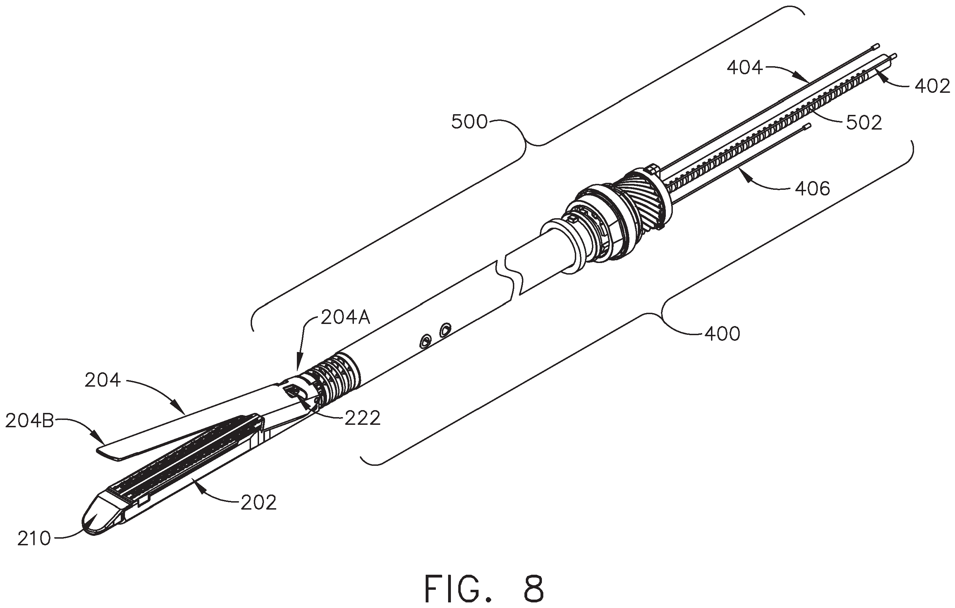

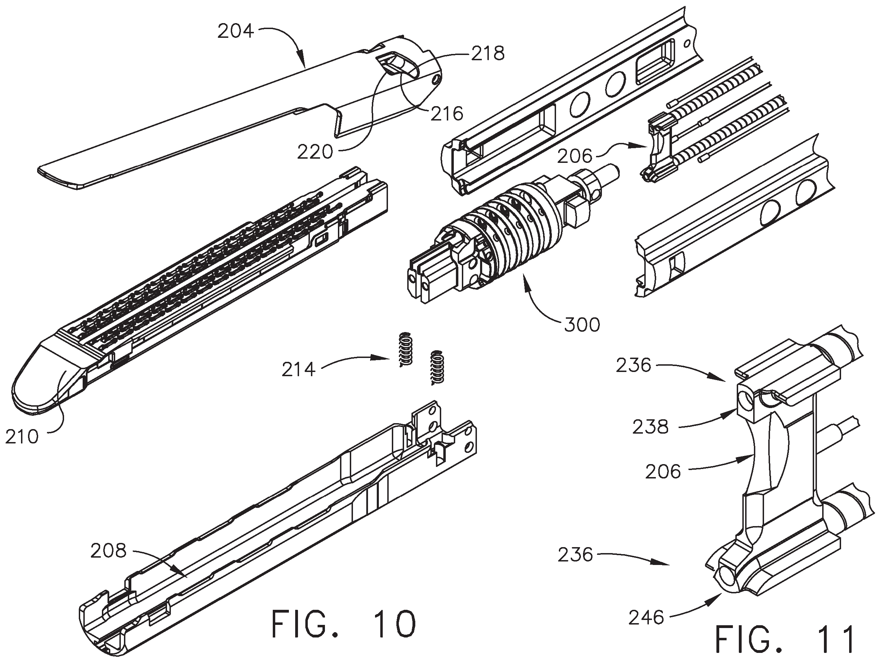

Like the surgical instrument 100, the surgical instrument 1000 may grasp, clamp, incise, and seal patient tissue. The surgical instrument 1000 includes an end effector 1200, an articulation joint 1300, an articulation assembly 1400 (FIG. 8) that articulates the end effector 1200 about the articulation joint 1300, and a firing assembly 1500 (FIG. 10) that moves the end effector 1200 to incise and staple patient tissue.

The surgical instrument 1000 further comprises a housing 1700 (FIGS. 7 and 7A) and a shaft 1600 extending from the housing 1700. The housing is configured to engage the robotic platform 2000. controlled by a clinician. To control the aforementioned assemblies 1400, 1500, respective proximal mechanisms are provided that interface with the robotic platform 2000. In some embodiments, the housing 1700 may be configured as a handle (e.g., it may comprise a grip for a clinician).

In at least one embodiment, the control circuit 12 may be incorporated into the housing 1700. In another embodiment, the control circuit 12 may be incorporated into the robotic platform 2000.

The housing 700 further comprises (1) a firing puck 1712A as part of the firing assembly 1500 operable to close the end effector 1200, fire staples, and transect tissue, (2) a set of articulation pucks 1702A, 1704A, 1706A, 1708A as part of the articulation assembly 1400 operable to articulate the end effector 1200 relative to the shaft 1600, and (3) a shaft roll puck 1710.

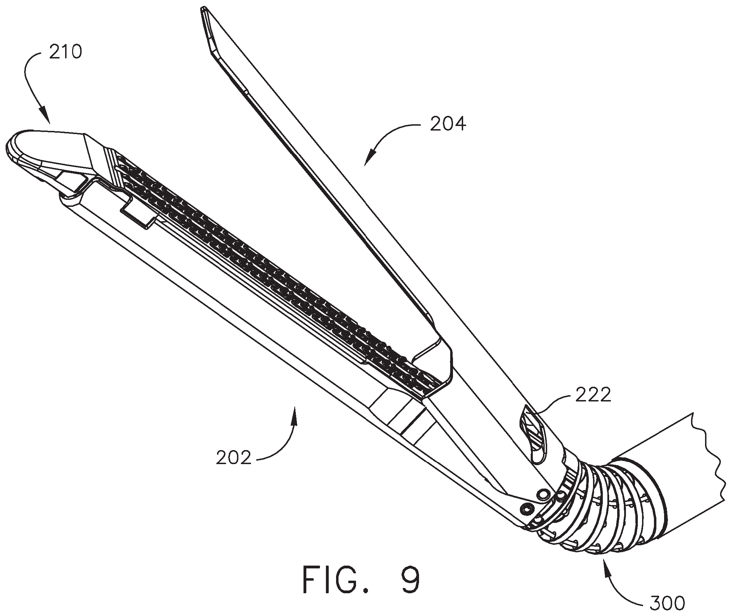

FIG. 9 illustrates the end effector 1200 at a desired articulation position with respect to the articulation joint based on user input received by the control circuit 12, according to at least one aspect of the present disclosure. The articulation joint 300 comprises a plurality of concentric discs and a center beam assembly. The articulation cables 1402, 1404, 1406, 1408 are routed and coupled to the end effector 1200 via the articulation joint 1300 such that movement thereof in a proximal direction causes the end effector 1200 to pivot in a predetermined manner about the articulation joint 1300. For example, actuation of the first articulation cable 1402 in the proximal direction causes rotation of the end effector 1200 upwards and to the left, actuation of the second articulation cable 404 in the proximal direction causes rotation of the end effector 1200 upwards and to the right, actuation of the third articulation cable 1406 in the proximal direction causes rotation of the end effector 1200 downwards and to the left, and actuation of the fourth articulation cable 1408 in the proximal direction causes rotation of the end effector 1200 downwards and to the right. Similarly, movement of two articulation cables simultaneously will result in blended movement of the end effector 1200. By way of example, movement of both the first articulation cable 1402 and the second articulation cable 1404 at the same rate causes only upwards pivoting of the end effector 1200 (i.e., there is little to no horizontal component to the rotation). As will be appreciated by those skilled in the art, this configuration provides for the above-mentioned precise 360-degree movement of the end effector about the articulation joint 1300 with at least two degrees of freedom and about 1320 degrees of roll.

Referring primarily to FIG. 7A, the robotic platform 2000 comprises a user interface 2005, a robot 2003, and an instrument interface 2001, which is designed to releasably couple to the surgical instrument 1000. In some embodiments, the instrument interface 2001 includes a series of pucks 2102A-2112A, which facilitate coupling with corresponding pucks 1702A-1712A on the housing 1700 of the surgical instrument 1000.

The robotic platform 2000 further includes a motor assembly 2002, which houses multiple motors that are operably linked to the pucks 2102A-2112A. These motors drive the transmission of motion to the pucks 1702A, 1704A, 1706A, 1708A, 1710A, and 1712A, thereby enabling articulation of the end effector 1200 and facilitating stapling and/or tissue transection functions. For example, dedicated articulation motors within the motor assembly 2002 generate controlled articulation movements, which are transmitted via cables 402, 404, 406, and 408 to adjust the orientation of the end effector 1200 to a desired articulation position, as illustrated in FIG. 9.

Furthermore, the control circuit 12 may adjust articulation of the end effector 1200, in a similar manner to that described in connection with the end effector 102, incorporating feedback mechanisms into operation of the robotic platform 2000. In at least one embodiment, control circuit 12 may continuously monitor the articulation state and tissue interaction forces, and utilizing real-time data for dynamic compensation of de-articulation forces, ensuring stable and consistent performance during critical surgical maneuvers, as described in connection with the instrument 100.

The end effector 1200 includes a first jaw 1202 and a second jaw 1204 movable between an open position and a closed position. For clarity, first jaw 1202 is herein also interchangeably used with "jaw 1202" (which is also referred to in the art as a "channel") and second jaw 1204 is used interchangeably with "anvil 1204". The jaw 1202 and anvil 1204 may be elongated in form. The jaw 1202 defines an elongated channel 1208 for receiving a staple cartridge 1210. The anvil 1204 has a proximal end 1204A, a distal end 1204B, and a ramp surface 1216 defined at the proximal end 1204A.

The jaw 1202 and anvil 1204 are pivotally coupled via a pivot pin 1212 that extends through the jaw 1202 and the anvil 1204. One or more biasing springs 1214 extend between the jaw 1202 and anvil 204 to bias the anvil 204 to the open position.

The surgical instrument 1000 further includes a firing assembly 1500 operable to close the anvil 1204 during a closure stroke. The firing assembly 1500 is operable to incise and staple, with staples from the staple cartridge 1210, the patient tissue captured between the staple cartridge 1210 (which is retained by the jaw 1202) and anvil 1204 during a firing stroke.

Referring primarily to FIG. 10 and 11, the firing assembly 1500 includes a knife 1206, a sled 1236, a firing rod 1502 that drives the knife 1206 and/or sled 1236. The firing rod 1502 includes a firing rod rack 1530 and is driven by a firing puck 1712A in the housing 1700.

The knife 1206 is coupled to or integral with a knife sled 1236. The knife sled 1236 is the non-cutting element of the knife 1206 and is also referred to as an I-beam. The knife sled 1236 includes an upper knife tab 1238 and a lower knife tab 1246. The upper knife tab 1238 is movable relative to the upper knife channel 1224 to move the anvil 1204 between the open position, the grasping position, and the clamping position.

The staple cartridge 1210 comprises a cartridge body. In use, the staple cartridge is positioned on a first side of the tissue to be stapled, within the channel 1208 of the jaw 1202, and the anvil 1204 is positioned on a second side of the tissue. The anvil 1204 is moved toward the staple cartridge 1210 to compress and clamp the tissue against the deck of the staple cartridge 1210. Thereafter, staples removably stored in the cartridge body can be deployed into the tissue. The cartridge body includes staple cavities defined therein wherein staples are removably stored in the staple cavities. In some embodiments, the staple cavities are arranged in six longitudinal rows. In some embodiments, three rows of staple cavities are positioned on a first side of a lower knife channel 1230 and three rows of staple cavities are positioned on a second side of lower knife channel 1230.

The staples are supported by staple drivers in the cartridge body. The drivers are movable between a first, or unfired position, and a second, or fired, position to eject the staples from the staple cavities. The drivers are retained in the cartridge body by a retainer which extends around the bottom of the cartridge body and includes resilient members configured to grip the cartridge body and hold the retainer to the cartridge body. The drivers are movable between their unfired positions and their fired positions indirectly by the sled 236. More specifically, the knife sled 1236 is movable between a proximal position adjacent the proximal end and a distal position adjacent the distal end. A portion of the knife sled 1236 engages a cartridge sled that slides under the drivers and lifts the drivers, and the staples supported thereon, toward the anvil 1204. It is desirable for the knife 1206 to be positioned at least partially proximal to the ramped surfaces such that the staples are in the second, or fired, position (i.e., ejected) ahead of the knife 1206.

Further to the above, the sled 1236 is moved distally and proximally by a firing rod 1502. The firing rod 1502 may apply an indirect force to the sled 1236 to push the sled 1236 toward the distal end of the end effector 200. As the firing rod 1502 is advanced distally, sled 1236 rides in the lower knife channel 1230 and the upper knife channel 1224. This movement first urges the anvil 1204 closed to a position where a compressive force is applied to the tissue sufficient to grasp it (referred to as the grasping position). Continued movement of the sled 1236 up the ramp surface 1216 results in a compressive force being applied to the tissue (referred to as the clamping position). As the anvil ramp surface 2116 transitions to its substantially horizontally angled surface, the upper knife tab 1238 can slide within the upper knife channel 1224 to drive the stapling and transection of the tissue. After the sled 1236 reaches the end of the transection path/line in the lower knife channel 230 (i.e., near a distal end thereof), the firing assembly 1500 then retracts the sled 1236 to a home, or starting position. The firing rod 1502 may include a firing rod rack 1530 (FIG. 8) driven by a firing puck assembly 1712 of the robotic platform 2000 to advance and/or retract the sled 1236.

In other embodiments, the surgical system 10 may be utilized with, or incorporated into, a surgical instrument or a surgical robotic system where closure and firing are executed separately and/or where knife motion does not directly drive closure/grasping. Various suitable mechanisms for separate closure and firing are disclosed in U.S. Patent Application No. 17/957,954, titled Monitoring one drive system to adapt the motor driven aspect of a second drive system, filed September 30, 2022, which is hereby incorporated by reference herein in its entirety.

Examples of the devices, systems, and methods disclosed herein, according to various aspects of the present disclosure, are provided below in the following numbered clauses. An aspect of the devices, systems, and methods may include any one or more than one, and any combination of, the numbered clauses described below.

Clause 1. A surgical system for use with a surgical instrument including an end effector articulatable relative to a shaft of the surgical instrument, the surgical system comprising: a control circuit configured to: receive an input signal indicative of a desired articulation position of the end effector; generate a drive signal for articulation of the end effector based on the input signal to the desired articulation position; and actively maintain the end effector at the desired articulation position based on a parameter independent of the articulation of the end effector.

Clause 2. The surgical system of Clause 1, wherein to actively maintain the end effector at the desired articulation, the control circuit is configured to: receive a sensor signal indicative of the parameter; and based on the sensor signal, dynamically compensate for an unintended de-articulation from the desired articulation position during a tissue treatment by the end effector.

Clause 3. The surgical system of Clause 1, wherein to actively maintain the end effector at the desired articulation position, the control circuit is further configured to: receive a sensor signal indicative of the parameter; and based on the sensor signal, generate an articulation control signal to dynamically adjust the articulation of the end effector during a tissue treatment by the end effector.

Clause 4. The surgical system of Clause 1, wherein the parameter indicates at least one of a torque, a force, or a load, associated with one or more components of a firing assembly configured to effect a tissue treatment by the end effector, and wherein the one or more components include at least one of a knife, a firing motor, a firing drive, or a firing rod.

Clause 5. The surgical system of Clause 4, wherein the parameter is a first parameter, and wherein the control circuit is configured to: actively maintain the end effector at the desired articulation position based on the first parameter and a second parameter, different than the first parameter, wherein the second parameter is independent of the articulation of the end effector.

Clause 6. The surgical system of Clause 5, wherein the second parameter indicates at least one of a distance or a position, associated with the one or more components of the firing assembly.

Clause 7. The surgical system of Clause 5, further comprising: at least one first sensor configured to determine the first parameter, wherein the first sensor is selected from a group including one or more of a strain-gauge sensor, a piezoelectric sensor, a current sensor, a force sensor, a torque sensor, or a load sensor.

Clause 8. The surgical system of Clause 7, further comprising: at least one second sensor configured to determine the second parameter, wherein the second sensor is selected from a group including one or more of a position sensor, a distance sensor, a Linear Variable Differential Transformer (LVDT), a rotary encoder, a potentiometer, a Hall Effect sensor, a capacitive sensor, an inductive sensor, or an optical sensor.

Clause 9. The surgical system of Clause 1, wherein to actively maintain the end effector at the desired articulation position, the control circuit is configured to: determine a maximum correction to the articulation of the end effector; determine a correction weight to the maximum correction; and dynamically adjust the articulation based on the maximum correction and the correction weight.

Clause 10. The surgical system of Clause 9, wherein the parameter is a first parameter, and wherein the control circuit is configured to: monitor the first parameter, wherein the maximum correction is based on the first parameter.

Clause 11.The surgical system of Clause 10, wherein the control circuit is configured to: monitor a second parameter, different than the first parameter, wherein the second parameter is independent of the articulation of the end effector, and wherein the correction weight is based on the second parameter.

Clause 12.The surgical system of Clause 11, wherein the correction weight is further based on a determined threshold associated with the second parameter.

Clause 13.The surgical system of Clause 1, wherein the parameter is associated with a knife movable relative to the end effector to transect tissue in a tissue treatment by the end effector, and wherein to actively maintain the end effector at the desired articulation position, the control circuit is further configured to: gradually increase, based on the parameter, a correction to the articulation for a first time interval associated with a start of the tissue transection by the knife.

Clause 14.The surgical system of Clause 13, wherein to actively maintain the end effector at the desired articulation position, the control circuit is further configured to:
gradually decrease, based on the parameter, the correction to the articulation for a second time interval associated with a start of a retraction of the knife relative to the end effector.

Clause 15. The surgical system of Clause 1, further comprising at least one articulation motor, wherein the drive signal causes the at least one articulation motor to articulate the end effector relative to the shaft based on the input signal.

Clause 16.A surgical system for use with a surgical instrument including an end effector articulatable relative to a shaft of the surgical instrument, the surgical system comprising: a control circuit configured to: receive an input signal indicative of a desired articulation position of the end effector; generate a drive signal for articulation of the end effector based on the input signal to the desired articulation position; receive a first sensor signal indicative of a knife input torque associated with advancing a knife relative to the end effector through a tissue transection path; receive a second sensor signal indicative of a position of the knife along the tissue transection path; and actively maintain the end effector at the desired articulation position based on the knife input torque and the position of the knife.

Clause 17.The surgical system of Clause 16, wherein to actively maintain the end effector at the desired articulation position, the control circuit is further configured to:
generate an articulation control signal to dynamically adjust the articulation of the end effector based on the knife input torque and the position of the knife.

Clause 18. The surgical system of Clause 17, wherein to dynamically adjust the articulation of the end effector, the control circuit is further configured to: determine a correction value to the articulation of the end effector based on the first sensor signal; determine a correction weight to the correction value based on the second sensor signal and a determined threshold; and generate the articulation control signal based on the correction value and the correction weight.

Clause 19.A surgical system, comprising: a surgical instrument, comprising: a shaft; an articulation joint extending from the shaft; an end effector articulatable about the articulation joint relative to the shaft; and a firing assembly, comprising a knife movable to cut tissue along a transection path defined by the end effector; and a control circuit configured to: receive a first sensor signal indicative of a knife input torque associated with advancing the knife relative to the end effector along the transection path; and generate an articulation control signal to actively maintain the end effector at a desired articulation position based on the knife input torque.

Clause 20. The surgical system of Clause 19, wherein the control circuit is configured to: receive a second sensor signal indicative of a position of the knife along the transection path, wherein the articulation control signal is further based on the position of the knife.

The systems, control circuits (e.g., control circuit 12) and methods, as described in connection with FIGS. 1-11 may include, or make use of, a number of computer apparatuses, computer systems, or the like. In other words, to utilize the systems and methods disclosed herein, at least one of a computer apparatus, computer system, or the like may be implemented.

As used in any aspect herein, the term "control circuit" may refer to, for example, hardwired circuitry, programmable circuitry (e.g., a computer processor including one or more individual instruction processing cores, processing unit, processor, microcontroller, microcontroller unit, controller, digital signal processor (DSP), programmable logic device (PLD), programmable logic array (PLA), or field programmable gate array (FPGA)), state machine circuitry, firmware that stores instructions executed by programmable circuitry, and any combination thereof. The control circuit may, collectively or individually, be embodied as circuitry that forms part of a larger system, for example, an integrated circuit (IC), an application-specific integrated circuit (ASIC), a system on-chip (SoC), desktop computers, laptop computers, tablet computers, servers, smart phones, etc. Accordingly, as used herein "control circuit" includes, but is not limited to, electrical circuitry having at least one discrete electrical circuit, electrical circuitry having at least one integrated circuit, electrical circuitry having at least one application specific integrated circuit, electrical circuitry forming a general purpose computing device configured by a computer program (e.g., a general purpose computer configured by a computer program which at least partially carries out processes and/or devices described herein, or a microprocessor configured by a computer program which at least partially carries out processes and/or devices described herein), electrical circuitry forming a memory device (e.g., forms of random access memory), and/or electrical circuitry forming a communications device (e.g., a modem, communications switch, or optical-electrical equipment). Those having skill in the art will recognize that the subject matter described herein may be implemented in an analog or digital fashion or some combination thereof.

In one embodiment, the control circuits, controllers or systems described herein may have a local data collection system deployed and may use machine learning to enable derivation-based learning outcomes. The control circuits may learn from and make decisions on a set of data (including data provided by various sensors), by making data-driven predictions and adapting according to the set of data. In embodiments, machine learning may involve performing a plurality of machine learning tasks by machine learning systems, such as supervised learning, unsupervised learning, and reinforcement learning. Supervised learning may include presenting a set of example inputs and desired outputs to the machine learning systems. Unsupervised learning may include the learning algorithm structuring its input by methods such as pattern detection and/or feature learning. Reinforcement learning may include the machine learning systems performing in a dynamic environment and then providing feedback about correct and incorrect decisions. In examples, machine learning may include a plurality of other tasks based on an output of the machine learning system. In examples, the tasks may be machine learning problems such as classification, regression, clustering, density estimation, dimensionality reduction, anomaly detection, and the like. In examples, machine learning may include a plurality of mathematical and statistical techniques. In examples, the many types of machine learning algorithms may include decision tree based learning, association rule learning, deep learning, artificial neural networks, genetic learning algorithms, inductive logic programming, support vector machines (SVMs), Bayesian network, reinforcement learning, representation learning, rule-based machine learning, sparse dictionary learning, similarity and metric learning, learning classifier systems (LCS), logistic regression, random forest, K-Means, gradient boost, K-nearest neighbors (KNN), a priori algorithms, and the like. In embodiments, certain machine learning algorithms may be used (e.g., for solving both constrained and unconstrained optimization problems that may be based on natural selection). In an example, the algorithm may be used to address problems of mixed integer programming, where some components restricted to being integer valued. Algorithms and machine learning techniques and systems may be used in computational intelligence systems, computer vision, Natural Language Processing (NLP), recommender systems, reinforcement learning, building graphical models, and the like. In an example, machine learning may be used making determinations, calculations, comparisons and behavior analytics, and the like.

One or more systems or assemblies, as described herein, employ one or more electric motors. In various forms, the electric motors may be a DC brushed driving motor, for example. In other arrangements, the motor may include a brushless motor, a cordless motor, a synchronous motor, a stepper motor, or any other suitable electric motor. The electric motors may be powered by a power source that in one form may comprise a removable power pack. Batteries may each comprise, for example, a Lithium Ion ("LI") or other suitable battery. The electric motors can include rotatable shafts that operably interface with gear reducer assemblies, for example. In certain instances, a voltage polarity provided by the power source can operate an electric motor in a clockwise direction wherein the voltage polarity applied to the electric motor by the battery can be reversed in order to operate the electric motor in a counter-clockwise direction. In various aspects, a microcontroller controls the electric motor through a motor driver via a pulse width modulated control signal. The motor driver can be configured to adjust the speed of the electric motor either in clockwise or counter-clockwise direction. The motor driver is also configured to switch between a plurality of operational modes which include an electronic motor braking mode, a constant speed mode, an electronic clutching mode, and a controlled current activation mode. In electronic braking mode, two terminal of the drive motor 200 are shorted and the generated back EMF counteracts the rotation of the electric motor allowing for faster stopping and greater positional precision.

Unless specifically stated otherwise as apparent from the foregoing disclosure, it is appreciated that, throughout the present disclosure, discussions using terms such as "processing," "computing," "calculating," "determining," "displaying," or the like, refer to the action and processes of a computer system, or similar electronic computing device, that manipulates and transforms data represented as physical (electronic) quantities within the computer system's registers and memories into other data similarly represented as physical quantities within the computer system memories or registers or other such information storage, transmission or display devices.

One or more components may be referred to herein as "configured to," "configurable to," "operable/operative to," "adapted/adaptable," "able to," "conformable/conformed to," etc. Those skilled in the art will recognize that "configured to" can generally encompass active-state components and/or inactive-state components and/or standby-state components, unless context requires otherwise.

Those skilled in the art will recognize that, in general, terms used herein, and especially in the appended claims (e.g., bodies of the appended claims) are generally intended as "open" terms (e.g., the term "including" should be interpreted as "including but not limited to," the term "having" should be interpreted as "having at least," the term "includes" should be interpreted as "includes but is not limited to," etc.). It will be further understood by those within the art that if a specific number of an introduced claim recitation is intended, such an intent will be explicitly recited in the claim, and in the absence of such recitation no such intent is present. For example, as an aid to understanding, the following appended claims may contain usage of the introductory phrases "at least one" and "one or more" to introduce claim recitations. However, the use of such phrases should not be construed to imply that the introduction of a claim recitation by the indefinite articles "a" or "an" limits any particular claim containing such introduced claim recitation to claims containing only one such recitation, even when the same claim includes the introductory phrases "one or more" or "at least one" and indefinite articles such as "a" or "an" (e.g., "a" and/or "an" should typically be interpreted to mean "at least one" or "one or more"); the same holds true for the use of definite articles used to introduce claim recitations.

In addition, even if a specific number of an introduced claim recitation is explicitly recited, those skilled in the art will recognize that such recitation should typically be interpreted to mean at least the recited number (e.g., the bare recitation of "two recitations," without other modifiers, typically means at least two recitations, or two or more recitations). Furthermore, in those instances where a convention analogous to "at least one of A, B, and C, etc." is used, in general such a construction is intended in the sense one having skill in the art would understand the convention (e.g., "a system having at least one of A, B, and C" would include but not be limited to systems that have A alone, B alone, C alone, A and B together, A and C together, B and C together, and/or A, B, and C together, etc.). In those instances where a convention analogous to "at least one of A, B, or C, etc." is used, in general such a construction is intended in the sense one having skill in the art would understand the convention (e.g., "a system having at least one of A, B, or C" would include but not be limited to systems that have A alone, B alone, C alone, A and B together, A and C together, B and C together, and/or A, B, and C together, etc.). It will be further understood by those within the art that typically a disjunctive word and/or phrase presenting two or more alternative terms, whether in the description, claims, or drawings, should be understood to contemplate the possibilities of including one of the terms, either of the terms, or both terms unless context dictates otherwise. For example, the phrase "A or B" will be typically understood to include the possibilities of "A" or "B" or "A and B."

With respect to the appended claims, those skilled in the art will appreciate that recited operations therein may generally be performed in any order. Also, although various operational flow diagrams are presented in a sequence(s), it should be understood that the various operations may be performed in other orders than those which are illustrated or may be performed concurrently. Examples of such alternate orderings may include overlapping, interleaved, interrupted, reordered, incremental, preparatory, supplemental, simultaneous, reverse, or other variant orderings, unless context dictates otherwise. Furthermore, terms like "responsive to," "related to," or other past-tense adjectives are generally not intended to exclude such variants, unless context dictates otherwise.

It is worthy to note that any reference to "one aspect," "an aspect," "an exemplification," "one exemplification," and the like means that a particular feature, structure, or characteristic described in connection with the aspect is included in at least one aspect. Thus, appearances of the phrases "in one aspect," "in an aspect," "in an exemplification," and "in one exemplification" in various places throughout the specification are not necessarily all referring to the same aspect. Furthermore, the features, structures or characteristics may be combined in any suitable manner in one or more aspects.

As used herein, the singular form of "a", "an", and "the" include the plural references unless the context clearly dictates otherwise.

Any patent application, patent, non-patent publication, or other disclosure material referred to in this specification and/or listed in any Application Data Sheet is incorporated by reference herein, to the extent that the incorporated materials is not inconsistent herewith. As such, and to the extent necessary, the disclosure as explicitly set forth herein supersedes any conflicting material incorporated herein by reference. Any material, or portion thereof, that is said to be incorporated by reference herein, but which conflicts with existing definitions, statements, or other disclosure material set forth herein will only be incorporated to the extent that no conflict arises between that incorporated material and the existing disclosure material. None is admitted to be prior art.

In summary, numerous benefits have been described which result from employing the concepts described herein. The foregoing description of the one or more forms has been presented for purposes of illustration and description. It is not intended to be exhaustive or limiting to the precise form disclosed. Modifications or variations are possible considering the above teachings. The one or more forms were chosen and described to illustrate principles and practical application to thereby enable one of ordinary skill in the art to utilize the various forms and with various modifications as are suited to the particular use contemplated. It is intended that the claims submitted herewith define the overall scope.

## Claims

1. A surgical system (10) for use with a surgical instrument (100) including an end effector (102) articulatable relative to a shaft (105) of the surgical instrument (100), the surgical system (10) comprising:
a control circuit (12) configured to:
receive an input signal indicative of a desired articulation position of the end effector (102);
generate a drive signal for articulation of the end effector (102) based on the input signal to the desired articulation position; and
actively maintain the end effector (102) at the desired articulation position based on a parameter independent of the articulation of the end effector (102).

2. The surgical system (10) of Claim 1, wherein to actively maintain the end effector (102) at the desired articulation position, the control circuit (12) is configured to:
receive a sensor signal indicative of the parameter; and
based on the sensor signal, dynamically compensate for an unintended de-articulation from the desired articulation position during a tissue treatment by the end effector (102).

3. The surgical system (10) of Claim 1, wherein to actively maintain the end effector (102) at the desired articulation position, the control circuit (12) is further configured to:
receive a sensor signal indicative of the parameter; and
based on the sensor signal, generate an articulation control signal to dynamically adjust the articulation of the end effector (102) during a tissue treatment by the end effector (102).

4. The surgical system (10) of any of Claims 1-3, wherein the parameter indicates at least one of a torque, a force, or a load, associated with one or more components of a firing assembly (110) configured to effect a tissue treatment by the end effector (102), and wherein the one or more components include at least one of a knife (103), a firing motor (104), a firing drive, or a firing rod (109).

5. The surgical system (10) of Claim 4, wherein the parameter is a first parameter, and wherein the control circuit (12) is configured to:
actively maintain the end effector (102) at the desired articulation position based on the first parameter and a second parameter, different than the first parameter, wherein the second parameter is independent of the articulation of the end effector (102).

6. The surgical system (10) of Claim 5, wherein the second parameter indicates at least one of a distance or a position, associated with the one or more components of the firing assembly (110).

7. The surgical system (10) of Claim 4 or 5, further comprising:
at least one first sensor (11) configured to determine the first parameter, wherein the first sensor (11) is selected from a group including one or more of a strain-gauge sensor, a piezoelectric sensor, a current sensor (13), a force sensor, a torque sensor, or a load sensor.

8. The surgical system (10) of Claim 7, further comprising:
at least one second sensor (11) configured to determine the second parameter, wherein the second sensor (11) is selected from a group including one or more of a position sensor, a distance sensor, a Linear Variable Differential Transformer (LVDT), a rotary encoder, a potentiometer, a Hall Effect sensor, a capacitive sensor, an inductive sensor, or an optical sensor.

9. The surgical system (10) of any of Claims 1-8, wherein to actively maintain the end effector (102) at the desired articulation position, the control circuit (12) is configured to:
determine a maximum correction to the articulation of the end effector (102);
determine a correction weight to the maximum correction; and
dynamically adjust the articulation based on the maximum correction and the correction weight.

10. The surgical system (10) of Claim 9, wherein the parameter is a first parameter, and wherein the control circuit (12) is configured to:
monitor the first parameter, wherein the maximum correction is based on the first parameter.

11. The surgical system (10) of Claim 10, wherein the control circuit (12) is configured to:
monitor a second parameter, different than the first parameter, wherein the second parameter is independent of the articulation of the end effector (102), and wherein the correction weight is based on the second parameter.

12. The surgical system (10) of Claim 11, wherein the correction weight is further based on a determined threshold associated with the second parameter.

13. The surgical system (10) of any of Claims 1-12, wherein the parameter is associated with a knife (103) movable relative to the end effector (102) to transect tissue in a tissue treatment by the end effector (102), and wherein to actively maintain the end effector (102) at the desired articulation position, the control circuit (12) is further configured to:
gradually increase, based on the parameter, a correction to the articulation for a first time interval associated with a start of the tissue transection by the knife (103).

14. The surgical system (10) of Claim 13, wherein to actively maintain the end effector (102) at the desired articulation position, the control circuit (12) is further configured to:
gradually decrease, based on the parameter, the correction to the articulation for a second time interval associated with a start of a retraction of the knife (103) relative to the end effector (102).

15. The surgical system (10) of any of Claims 1-14, further comprising at least one articulation motor, wherein the drive signal causes the at least one articulation motor to articulate the end effector (102) relative to the shaft (105) based on the input signal.
